# EUROPEAN PATENT SPECIFICATION

(11) **EP 3 791 842 B1**
(45) Date of publication and mention of the grant of the patent: **03.09.2025**
(21) Application number: 19827006.8
(22) Date of filing: 18.06.2019
(51) Int. Cl.: A61F 13/494, A61F 13/511, A61F 13/53, A61F 13/49

(54) **ABSORBENT ARTICLE**
ABSORBIERENDER ARTIKEL
ARTICLE ABSORBANT

(30) Priority: 29.06.2018 JP 2018124776
(43) Date of publication of application: 17.03.2021
(73) Proprietor: Unicharm Corporation, Ehime 799-0111 (JP)
(72) Inventor: NAGAI, Takahito, Kanonji-shi, Kagawa 769-1602 (JP); TANAKA, Suguru, Kanonji-shi, Kagawa 769-1602 (JP)
(74) Representative: Dolleymores
(86) International application number: PCT/JP2019/024135
(87) International publication number: WO 2020/004151

(56) References cited:
- JP-A- 2008 302 138
- JP-A- 2009 000 512
- JP-A- 2014 180 345
- JP-A- 2018 000 435

## Description

### [Technical Field]

The present invention relates to an absorbent article.

### [Background Art]

As underpants-shaped absorbent articles, underpants-shaped disposable diapers are known. Patent Document 1 discloses an underpants-shaped disposable diaper including an outer sheet member, an absorbent body joined to a skin surface side of the outer sheet member, and side sheets respectively extending from two widthwise end portions of the absorbent body. In the diaper of Patent Literature 1, raising elastic members (38 and 39) for raising the side sheets are attached in a stretched state to the side sheets throughout the entire length of the side sheets in the longitudinal direction of the absorbent body. In addition, the side sheets rise toward the skin side of a wearer due to the contraction of the raising elastic members and function as so-called leakproof walls.

### [Patent Literature]

Patent Literature 1: Japanese Patent Application Publication No. 2010-94334
EP 3441053 A1 discloses an absorbent article comprising an absorbent main body, a pair of side flaps and a pair of leakage barriers. Each of the pair of leakage barriers includes a standing portion, a pair of supporting portions and a joining portion. The leakage barriers are fixed to the absorbent main body with a heat welding portion.

### [Summary of the Invention]

### [Technical Problem]

In the diaper of Patent Literature 1, fixing portions (joining portions 41c) that fix the side sheets to a top sheet of the absorbent body are provided at longitudinal middle portions of the side sheets (leakproof walls). Such fixing portions are generally formed using an adhesive such as a hot melt adhesive. However, in a case where the fixing portions are disposed in the vicinity of the crotch portion of the wearer (an intermediate portion of the absorbent body in the front-back direction) as in Patent Literature 1, the fixing portions are likely to peel off due to the influence of friction or wetting, and it is difficult to firmly join the absorbent body (top sheet) and the side sheets (leakproof walls).

The present invention was achieved in light of conventional problems such as that described above, and an aspect of the present invention is to provide an absorbent article whose intermediate fixing portion for joining a leakproof wall portion and an absorbent body at an intermediate portion in a front-back direction does not easily peel off.

### [Solution to Problem]

The present invention provides the absorbent article of independent claim 1. The dependent claims specify preferred but optional features.

A main aspect of the present invention for achieving the above-described aspect is an absorbent article including: an absorbent body including an absorbent core and a hydrophilic skin-side sheet disposed on a skin side with respect to the absorbent core; and a pair of leakproof wall portions, in a state in which the absorbent article is unfolded, the absorbent body having a front-back direction, a lateral direction, and a thickness direction that intersect each other, the pair of leakproof wall portions being provided on two lateral sides of the absorbent body, a front fixing portion being provided in a front end portion of the absorbent body and fixing the leakproof wall portions so that the leakproof wall portions are incapable of rising, a back fixing portion being provided in a back end portion of the absorbent body and fixing the leakproof wall portions so that the leakproof wall portions are incapable of rising, an intermediate fixing portion being provided between the front fixing portion and the back fixing portion in the front-back direction, the intermediate fixing portion joining the leakproof wall portion and the absorbent body at an intermediate portion in the front-back direction, the intermediate fixing portion being provided apart from the front fixing portion and the back fixing portion in the front-back direction, the intermediate fixing portion fixing the leakproof wall portions to the skin-side sheet, in the intermediate fixing portion, a predetermined adhesive being provided between the skin-side sheet and the leakproof wall portions, in the intermediate fixing portion, the skin-side sheet being impregnated with at least a part of the adhesive, the skin-side sheet having a plurality of compressed portions in which the skin-side sheet is compressed in the thickness direction, a portion in which the adhesive and the compressed portions overlap in the thickness direction is provided. Other features of the present invention will be further clarified by the description of the present specification and the accompanying drawings.

### [Advantageous Effect of the Invention]

According to the present invention, it is possible to provide an absorbent article whose intermediate fixing portion for joining a leakproof wall portion and an absorbent body at an intermediate portion in a front-back direction does not easily peel off.

### [Brief Description of Drawings]

FIG. 1 is a schematic front view of a diaper 1.
FIG. 2 is a schematic plan view of the diaper 1 in an unfolded and stretched state.
FIG. 3 is a schematic cross-sectional view taken along a line I-I in FIG. 2.
FIG. 4A and FIG. 4B are diagrams illustrating disposition examples of compressed portions 60 provided on a skin-side sheet 12.
FIG. 5 is a schematic cross-sectional view showing a state in which an intermediate fixing portion 53 is provided on a skin-side surface of the skin-side sheet 12 in the diaper 1.
FIG. 6 is a schematic cross-sectional view showing a state in which the intermediate fixing portion 53 is provided on the skin-side surface of the skin-side sheet 12 in a comparative example.
FIG. 7 is a schematic cross-sectional view illustrating how the intermediate fixing portion 53 of the comparative example peels off from the skin-side sheet 12.

### [Description of Embodiments]

Description of the present specification and the accompanying drawings clarifies at least the following matters.

An absorbent article including: an absorbent body including an absorbent core and a hydrophilic skin-side sheet disposed on a skin side with respect to the absorbent core; and a pair of leakproof wall portions, in a state in which the absorbent article is unfolded, the absorbent body having a front-back direction, a lateral direction, and a thickness direction that intersect each other, the pair of leakproof wall portions being provided on two lateral sides of the absorbent body, a front fixing portion being provided in a front end portion of the absorbent body and fixing the leakproof wall portions so that the leakproof wall portions are incapable of rising, a back fixing portion being provided in a back end portion of the absorbent body and fixing the leakproof wall portions so that the leakproof wall portions are incapable of rising, an intermediate fixing portion being provided between the front fixing portion and the back fixing portion in the front-back direction, the intermediate fixing portion fixing the leakproof wall portions to the skin-side sheet, in the intermediate fixing portion, a predetermined adhesive being provided between the skin-side sheet and the leakproof wall portions, in the intermediate fixing portion, the skin-side sheet being impregnated with at least a part of the adhesive, the skin-side sheet having a plurality of compressed portions in which the skin-side sheet is compressed in the thickness direction, a portion in which the adhesive and the compressed portions overlap in the thickness direction is provided.

According to the above-described absorbent article, the adhesive that forms the intermediate fixing portion is integrally fixed with the compressed portions provided on the skin-side sheet. This makes the joining strength strong, making it less likely for the intermediate fixing portion to peel off from the skin-side sheet. Therefore, even when there is an influence of friction with the body of wearers or wetting caused by excrement such as urine while the absorbent article is put on, it becomes easy to maintain the joining between the leakproof wall portions and the absorbent body.

In such an absorbent article, it is desirable that,
in the thickness direction,
the adhesive overlaps a skin-side end of the compressed portion and
the adhesive does not overlap a non-skin-side end of the compressed portion.

According to the above-described absorbent article, in the thickness direction, at least the non-skin side end of the skin-side sheet is not impregnated with the adhesive. This suppresses the skin-side sheet becoming excessively hard, making it easier to maintain soft texture of the absorbent body. This can make it difficult to cause discomfort to wearers while the absorbent article is put on.

In such an absorbent article, it is desirable that
the adhesive overlaps the entire compressed portion in the thickness direction.

According to the above-described absorbent article, the entire compressed portions are integrally fixed with the adhesive in the thickness direction. This makes stronger the joining strength between the intermediate fixing portion and the skin-side sheet. Therefore, even in a case where a large load (friction) is applied to the intermediate fixing portion or even in a case where the intermediate fixing portion frequently comes into contact with excrement such as urine, it is easy to maintain the joining between the leakproof wall portions and the absorbent body.

In such an absorbent article, it is preferable
that a core wrap sheet that covers the absorbent core is provided, and
that the adhesive has a portion that overlaps the core wrap sheet in the thickness direction.

According to the above-described absorbent article, it is possible to join the skin-side sheet and the core wrap sheet that is stacked in the thickness direction on the skin-side sheet, fixing the core wrap sheet. This makes less likely to cause the shape collapse and/or positional deviation of the absorbent core covered with the core wrap sheet, making it possible to enhance the fitting property and/or absorption performance of the absorbent body.

In such an absorbent article, it is desirable that
in the thickness direction, the adhesive does not overlap a portion of the skin-side sheet that is disposed on a non-skin side with respect to the absorbent core.

According to the above-described absorbent article, the adhesive is suppressed from exuding outward in the lateral direction from a portion of the skin-side sheet that is disposed on the non-skin side from the absorbent core. Therefore, it is possible to suppress the adhesive adhering to the body and/or clothing of wearers.

In such an absorbent article, it is desirable that
the adhesive does not overlap skin-side surfaces of the leakproof wall portions in the thickness direction.

According to the above-described absorbent article, it is possible to suppress the adhesive- impregnated depth from reaching the skin-side surfaces of the leakproof wall portions, and the adhesive exuding from the skin-side surfaces of the leakproof wall portions is prevented from adhering the body of wearers.

In such an absorbent article, it is desirable that
a lateral width of the intermediate fixing portion is larger than a lateral pitch between the plurality of compressed portions.

According to the above-described absorbent article, even in a case where a force acts in a direction in which the intermediate fixing portion is peeled off from the skin-side sheet, the two or more compressed portions make it possible to resist the force that peels off the intermediate fixing portion. This can further increase the joining strength between the skin-side sheet and the intermediate fixing portion.

In such an absorbent article, it is desirable that
in the lateral direction, a predetermined gap is provided between an outer end of the intermediate fixing portion and an outer end of the leakproof wall portion.

According to the above-described absorbent article, a space is formed at the lateral outer end portion (edge portion) of the leakproof wall portion. This reduces the hardness of the edge portion, and makes soft the texture of the absorbent article when coming into contact with the body (groin) of wearers while the absorbent article is put on. Therefore, it is possible to realize a more favorable fitting property.

In such an absorbent article, it is desirable that
in the lateral direction, a predetermined gap is provided between an inner end of the intermediate fixing portion and an inner end of the leakproof wall portion.

With the above-described absorbent article, the entire region of the intermediate fixing portion is covered with the leakproof wall portion in the lateral direction, making it less likely to expose an adhesive that forms the intermediate fixing portion to the outside. This can suppress the exposed adhesive adhering to the body or clothing of wearers while the absorbent article is put on.

In such an absorbent article, it is desirable
that the absorbent core has a narrow portion in a central part in the front-back direction,
   the narrow portion having a lateral width smaller than end portions of the absorbent core, and
that at least a part of the intermediate fixing portion is arranged side-by-side in the lateral direction with the narrow portion, and is disposed outside the narrow portion in the lateral direction.

According to the above-described absorbent article, the intermediate fixing portion and the absorbent core are disposed so as not to overlap, whereby a skin-side surface of the narrow portion being covered with the leakproof wall portions and the intermediate fixing portion is suppressed. Therefore, it is possible to suppress the absorption area of the absorbent core becoming smaller in the narrow portion and to facilitate the maintaining of the absorption capability of the absorbent core.

### Embodiment

Hereinafter, embodiments will be described using an underpants-shaped disposable diaper for adults (hereinafter also referred to as "diaper 1"), as an absorbent article according to the present invention. However, the absorbent article according to the present invention is also available as disposable diapers for infants, sanitary shorts, and the like.

### Configuration of diaper 1

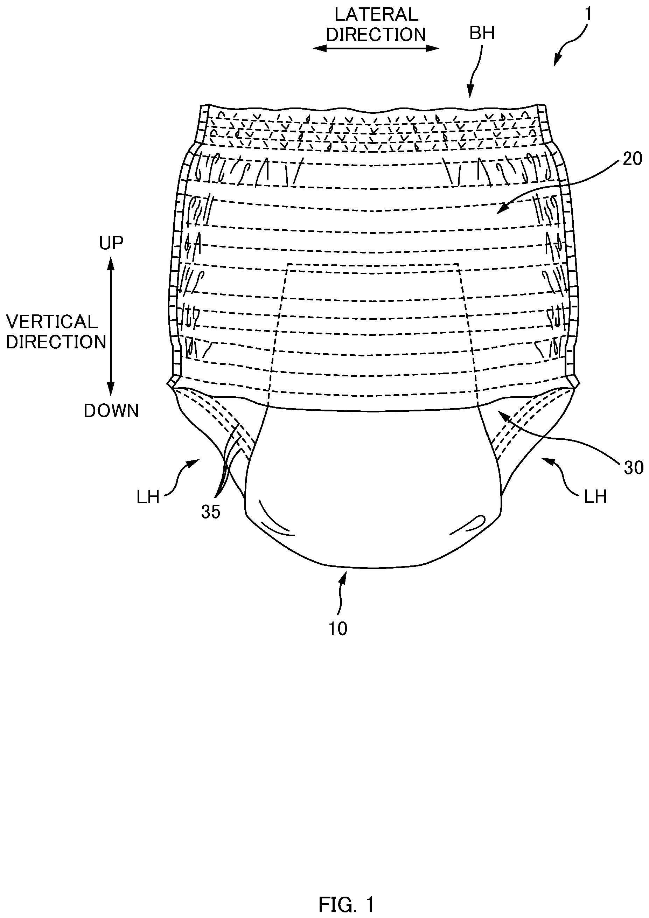

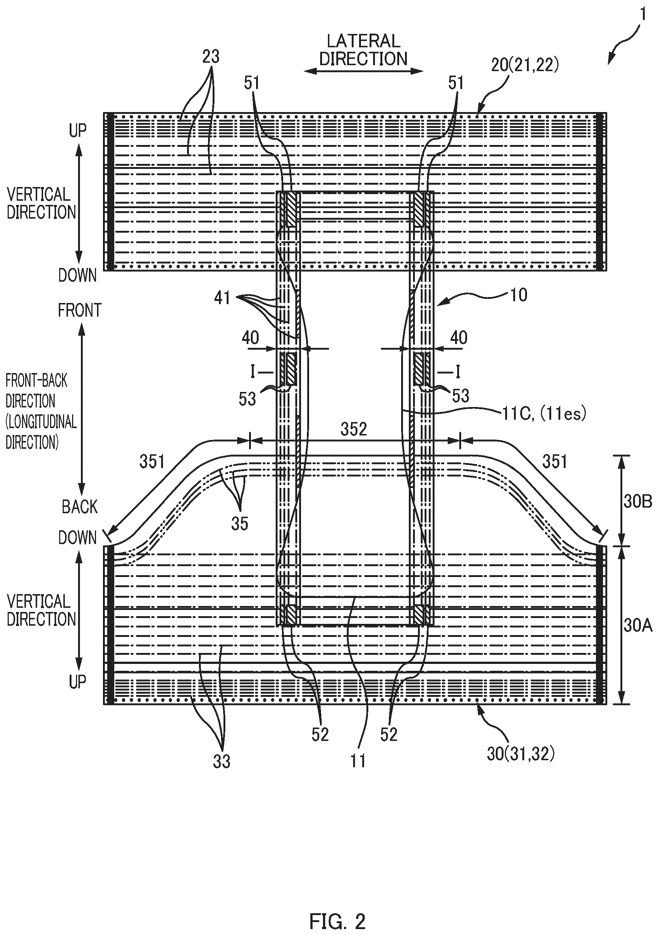

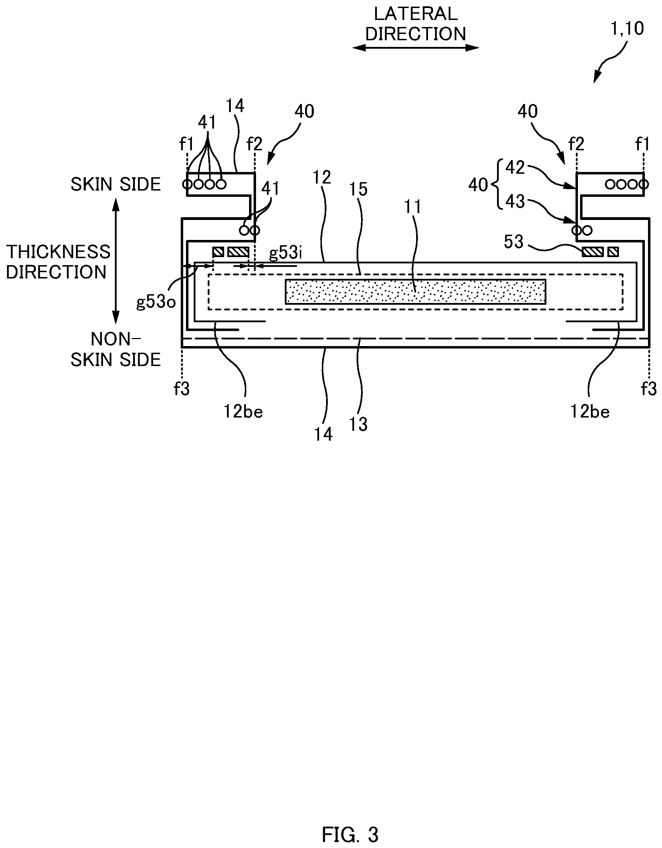

FIG. 1 is a schematic front view of the diaper 1. FIG. 2 is a schematic plan view of the diaper 1 in an unfolded and stretched state. FIG. 3 is a schematic cross-sectional view taken along a line I-I in FIG. 2. It should be noted that the "stretched state" of the diaper 1 refers to a state in which the entire diaper 1 (the entire product) is stretched without forming wrinkles, specifically, a state in which the diaper is stretched such that the dimensions of constituent members of the diaper 1 (e.g., an absorbent body 10, waist portions 20 and 30, or the like that will be described later) match or are close to the dimensions of the members on their own.

In an underpants-shaped state shown in FIG. 1, the diaper 1 has a vertical direction, a lateral direction, and a front-back direction that intersect with each other and has a waist opening BH and a pair of leg openings LH. The upper side in the vertical direction corresponds to the waist opening BH side, and the lower side corresponds to the crotch side. In addition, the front side in the front-back direction corresponds to the front side of a wearer, and the back side corresponds to the back side of the wearer. In addition, in an unfolded state of FIG. 2, the diaper 1 has the front-back direction and the lateral direction that intersect each other. The front-back direction corresponds to the longitudinal direction of the absorbent body 10. In addition, as shown in FIG. 3, a direction in which constituent members of the diaper 1 are stacked is referred to as a thickness direction. In the thickness direction, a side that comes into contact with the wearer is defined as a skin side, and a side opposite to the skin side is defined as a non-skin side.

The diaper 1 has an absorbent body 10, a front waist portion 20, and a back waist portion 30. In the diaper 1 in the underpants-shaped state, the absorbent body 10 (an absorbent core 11 described below) is disposed extending along the vertical direction and is folded in the front-back direction, at the lower end portion in the vertical direction. In addition, the front waist portion 20 is joined to the non-skin side of the front upper end portion of the absorbent body 10, and the back waist portion 30 is joined to the non-skin side of the back upper end portion of the absorbent body 10.

In the diaper 1 in the unfolded state of FIG. 2, the front waist portion 20 and the back waist portion 30 are disposed such that the longitudinal directions thereof are along the lateral direction of the diaper 1. In addition, the longitudinal one-side (front) end portion of the absorbent body 10 is disposed in the lateral central part of the front waist portion 20, and the longitudinal other-side (back) end portion of the absorbent body 10 is disposed in the lateral central part of the back waist portion 30. In the unfolded state shown in FIG. 2, the absorbent body 10 is folded one time at substantially the center in the front-back direction (longitudinal direction), and two lateral side portions of the front waist portion 20 and two lateral side portions of the back waist portion 30 are respectively joined by welding or the like, thereby forming the diaper 1 in the underpants-shaped state.

The absorbent body 10 has the absorbent core 11 that absorbs excrement, a hydrophilic skin-side sheet 12 disposed on the wearer's skin side of the absorbent core 11, a liquid-impermeable non-skin-side sheet 13 disposed on the non-skin side of the absorbent core 11, and a sheet member 14. Two lateral side portions of the skin-side sheet 12 are folded back toward the non-skin side so as to enclose the absorbent core 11. The sheet member 14 is disposed on the non-skin side with respect to the non-skin-side sheet 13 and forms leakproof wall portions 40 on the skin side with respect to of the skin-side sheet 12.

The absorbent core 11 is an absorbent core obtained by shaping a liquid absorbent fiber such as a pulp fiber containing a superabsorbent polymer (SAP) into a predetermined shape. In addition, the absorbent core 11 may be covered with a liquid-permeable core wrap sheet 15.

In addition, the absorbent core 11 of the present embodiment has a narrow portion 11C in the central part in the front-back direction, and the narrow portion 11C is a portion where the lateral length of the absorbent core 11 is shorter (has a narrower width) compared with the front end portion and the back end portion. Accordingly, the absorbent core 11 has a substantially hourglass shape in a plan view as shown in FIG. 2. This narrow portion 11C is a portion sandwiched between both legs of the wearer while the diaper 1 is put on, and the lateral length thereof is small (the width is narrow), making the absorbent core 11 easier to fit to the crotch of the wearer.

The front waist portion 20 (the back waist portion 30) includes an inner-layer sheet 21 (31), an outer-layer sheet 22 (32) disposed on the non-skin side with respect to the inner-layer sheet 21 (31), and waist elastic members 23 (33). The waist elastic members 23 (33) are disposed side by side in the vertical direction between the inner-layer sheet 21 (31) and the outer-layer sheet 22 (32) and are fixed in a laterally stretched state. Therefore, the front waist portion 20 and the back waist portion 30 fit the waist of the wearer. In addition, a cover sheet (not shown) that covers the upper end portion of the absorbent body 10 from the skin side may also be provided.

In the unfolded and stretched state of FIG. 2, the front waist portion 20 has a rectangular shape in a plan view. The back waist portion 30 includes: an overlapping portion 30A that overlaps the front waist portion 20 in the front-back direction; and an extension portion 30B that extends downward beyond the front waist portion 20. The overlapping portion 30A has a rectangular shape in a plan view, and the extension portion 30B has an inverted trapezoidal shape in a plan view. The buttocks of the wearer can be covered with the extension portion 30B. The front waist portion 20 has a short vertical length compared with the back waist portion 30 and does not extend up to the crotch side. Therefore, the movement of legs that are pulled up to the front side is not hindered during walking or the like, and the wearer easily walks.

In addition, in the extension portion 30B of the back waist portion 30, extension-portion elastic members 35 are provided along the lower end of the extension portion 30B. the extension-portion elastic member 35 has: a pair of inclined portions 351 extending downward and inclined inward in the lateral direction; and a straight portion 352 extending along the lateral direction and between the pair of inclined portions 351. Similar to the waist elastic members 33, the extension-portion elastic members 35 are fixed in a stretched state, between the inner-layer sheet 31 and the outer-layer sheet 32. The extension-portion elastic members 35 fit the extension portion 30B to the buttocks of the wearer, making it possible to suppress the extension portion 30B being curling-up.

However, the configuration is not limited thereto. For example, the front waist portion 20 may have the same planar shape as the back waist portion 30 and may include the same elastic members as the extension-portion elastic members 35 in the back waist portion 30. That is, the front waist portion 20 may include inclined elastic members extending downward and inclined inward in the lateral direction, at the lower end portion. In addition, conversely, the back waist portion 30 may have the same rectangular shape in a plan view as the front waist portion 20 and may not include the extension-portion elastic members 35.

On two lateral side portions of the absorbent body 10, a pair of the leakproof wall portions 40 are respectively provided extending along the front-back direction (the longitudinal direction of the absorbent body 10). In the present embodiment, the pair of leakproof wall portions 40 is respectively formed by folding the sheet member 14 that constitutes the exterior of the absorbent body 10, at multiple positions as shown in FIG. 3. Specifically, the sheet member 14 is folded at folding positions f1 in FIG. 3 into two layers, and is further folded at folding positions f2 and folding positions f3. Thus, the leakproof wall portions 40 are formed being folded so as to have a substantially S-shaped cross-section.

In the leakproof wall portion 40, a portion between the folding lines f1 and f2 is defined as a "skin-side portion 42", and a portion between the folding lines f2 and f3, which is disposed on the non-skin side of the skin-side portion 42, is defined as a "non-skin-side portion 43". The skin-side portion 42 is a portion folded outward in the lateral direction relative to the non-skin-side portion 43.

In addition, in the leakproof wall portion 40, between the two overlaid layers of the sheet member 14, a predetermined stretching/contracting member is fixed in a state of being stretched in the front-back direction of the absorbent body 10 (corresponding to the vertical direction of the diaper 1). In the present embodiment, as the stretching/contracting member, a plurality of leakproof-wall stretching/contracting members 41 such as elastic strings are disposed side by side in the lateral direction. While the diaper 1 is put on, the stretchability generated by the leakproof-wall stretching/contracting members 41 (stretching/contracting member) makes the leakproof wall portions 40 contract along the front-back direction of the absorbent body 10 and rise toward the skin side of the wearer, fitting the diaper 1 to the crotch portion of the wearer.

It should be noted that the leakproof wall portions 40 are fixed by front fixing portions 51 provided at the front end portion, so as to be incapable of rising in the front end portion of the absorbent body 10 in the front-back direction. Similarly, the leakproof wall portions 40 are fixed by back fixing portions 52 provided at the back end portion, so as to be incapable of rising in the back end portion of the absorbent body 10 in the front-back direction (see FIG.2).

Therefore, the leakproof wall portions 40 become capable of rising toward the skin side of the wearer, in a region between the front fixing portions 51 and the back fixing portions 52 in the front-back direction of the absorbent body 10. In addition, for example, in a case where the wearer is in a lying-down posture, even when liquid excrement held by the leakproof wall portions 40 flows in the front-back direction, it is possible for the front fixing portions 51 and the back fixing portions 52 to block the liquid excrement, making it possible to suppress front leakage or back leakage.

In addition, between the front fixing portions 51 and the back fixing portions 52 in the front-back direction of the absorbent body 10, intermediate fixing portions 53 that fix the leakproof wall portions 40 to the skin-side sheet 12 of the absorbent body 10 are provided. As shown in FIG. 2, the intermediate fixing portions 53 of the present embodiment are provided apart from the front fixing portions 51 and the back fixing portions 52 in the front-back direction. That is, in the absorbent body 10, the front fixing portions 51, the intermediate fixing portions 53, and the back fixing portions 52 are intermittently disposed in the front-back direction in a state in which the diaper 1 is unfolded and stretched. It should be noted that all of the front fixing portions 51, the back fixing portions 52, and the intermediate fixing portions 53 are formed of an adhesive such as a hot melt adhesive.

In portions in the front-back direction where the intermediate fixing portions 53 are provided, since the non-skin-side portions 43 of the leakproof wall portions 40 are fixed to the skin-side sheet 12 (see FIG.3), only the skin-side portions 42 are capable of rising toward the skin side. That is, in the portions where the intermediate fixing portions 53 are provided, the height up to which the leakproof wall portions 40 are capable of rising is limited, and it is possible to decrease the gap between the absorbent body 10 and the skin of the wearer while the diaper 1 is put on. Accordingly, appropriately adjusting the disposition or size of the intermediate fixing portions 53 makes it possible to change the fitting property of the diaper 1 around the wearer's legs and/or the excrement absorption performance.

### Intermediate fixing portions 53

As described in FIG. 3, in the diaper 1, the skin-side surface of the absorbent body 10 (skin-side sheet 12) and the non-skin side surfaces of the leakproof wall portions 40 (sheet member 14) are joined with the intermediate fixing portions 53. Since these intermediate fixing portions 53 are positioned in the vicinity of the crotch part of the wearer while the diaper 1 is put on, the intermediate fixing portions 53 are likely to be affected by friction generated when the wearer moves the body and/or by wetting caused by urination (liquid). In addition, when the intermediate fixing portions 53 are affected as described above, there is a risk that the intermediate fixing portions 53 peel off and the leakproof wall portions 40 detach from the absorbent body 10.

In addition, conventionally, in the case of joining the skin-side sheet 12 and the leakproof wall portions 40 as described above, it is difficult to obtain a sufficient joining strength, and the intermediate fixing portions 53 are likely to peel off at the interface with the skin-side sheet 12. This reason is as follow. The skin-side sheet 12 is formed of a spunbond nonwoven fabric and/or pointbond nonwoven fabric which are hydrophilic and contain an oil agent. This makes it difficult to obtain a sufficient joining strength only by interfacial joining between the adhesive that constitutes the intermediate fixing portion 53 and the surface fiber of the skin-side sheet 12 (hydrophilic nonwoven fabric).

It should be noted that the sheet member 14 that constitutes the leakproof wall portions 40 is formed of a hydrophobic spunbond-meltblown-spunbond (SMS) nonwoven fabric, film, or the like containing no oil agent, in order to suppress the exudation of urine or the like. Therefore, the strength of the interfacial joining between the adhesive that constitutes the intermediate fixing portion 53 and the sheet member 14 is stronger than the strength of the interfacial joining between the adhesive and the skin-side sheet 12, making it easier to obtain a sufficient joining strength between the intermediate fixing portion 53 and the leakproof wall portion 40.

In addition, in the diaper 1, the detachment of the leakproof wall portions 40 from the absorbent body 10 is suppressed by sufficiently increasing the joining strength between the intermediate fixing portion 53 and the skin-side sheet 12. Hereinafter, the structure of the intermediate fixing portion 53 in the present embodiment will be described.

First, the structure of the skin-side sheet 12 will be briefly described. The skin-side sheet 12 is a commercially available hydrophilic nonwoven fabric, and a plurality of compressed portions 60 is provided on the surface of the skin-side sheet 12. The compressed portions 60 are formed by compressing the skin-side sheet 12 from one side to the other side in the thickness direction using well-known compression means such as embossing. In places compressed by the compressed portions 60, the fiber density of the skin-side sheet 12 becomes partially high, increasing the rigidity.

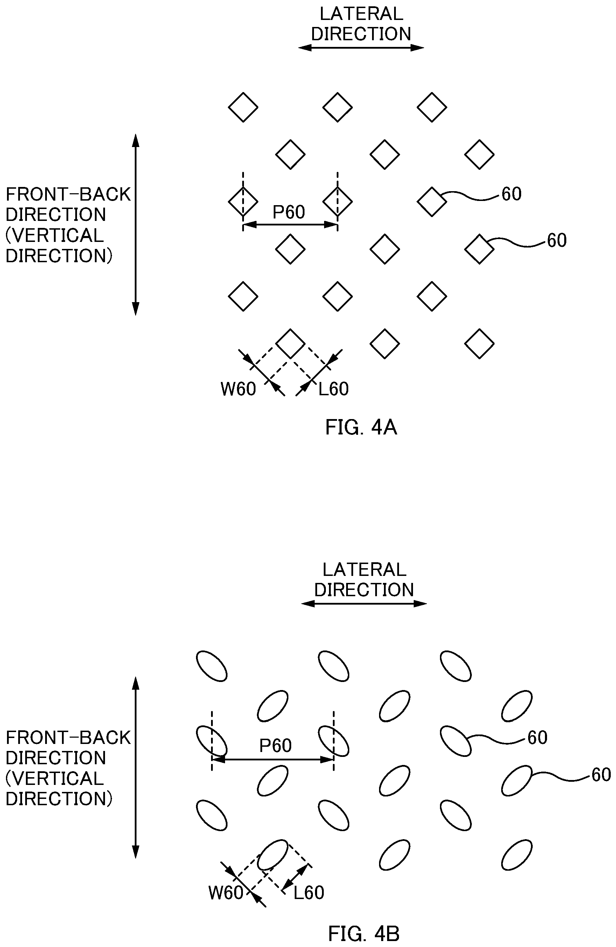

FIG. 4A and FIG. 4B are diagrams illustrating disposition examples of the compressed portions 60 provided on the skin-side sheet 12. In the example of FIG. 4A, a plurality of rectangular compressed portions 60 is disposed in a so-called zigzag disposition pattern. Regarding the size of each compressed portion 60, when the length of one side of the rectangular shape (defined as the first direction length) is represented by L60, and the length of a different side orthogonal to the above-described side (defined as the second direction length) is represented by W60, both length L60 and length W60 are approximately 0.4 mm or larger and 1.2 mm or smaller (0.4 mm ≤ L60, W60 ≤ 1.2 mm). In addition, the center-to-center distance (that is, the pitch in the lateral direction) P60 between two compressed portions 60 and 60 adjacent to each other in the lateral direction is approximately 1.5 mm or more and 3.0 mm or less (1.5 mm ≤ P60 ≤ 3.0 mm).

In the example of FIG. 4B, elliptical compressed portions 60 are disposed in a zigzag disposition pattern. When the length of the ellipse in the long-side direction (defined as the first direction length) is represented by L60, and the length of the ellipse in the short-side direction (defined as the second direction length) is represented by W60, both length L60 and length W60 are, similar to the case of FIG. 4A, 0.4 mm or larger and 1.2 mm or smaller (0.4 mm ≤ L60, W60 ≤ 1.2 mm). In addition, the lateral center-to-center distance (pitch) P60 is also, similar to the case of FIG. 4A, approximately 1.5 mm or more and 3.0 mm or less (1.5 mm ≤ P60 ≤ 3.0 mm). However, the shape and/or disposition pattern of the compressed portions 60 is not limited to these examples. For example, the compressed portion 60 may have a circular or polygonal shape, and the compressed portions 60 may be disposed in a grid-like or other disposition pattern.

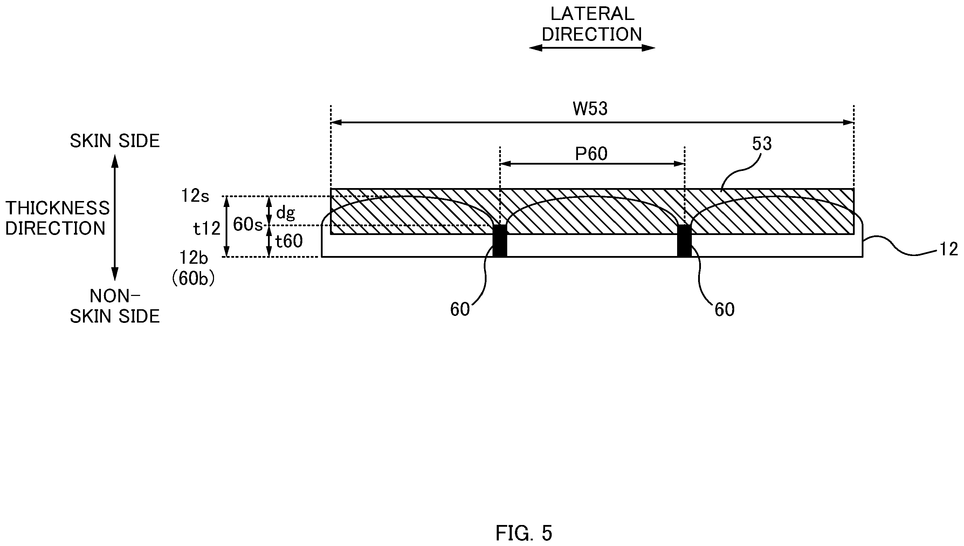

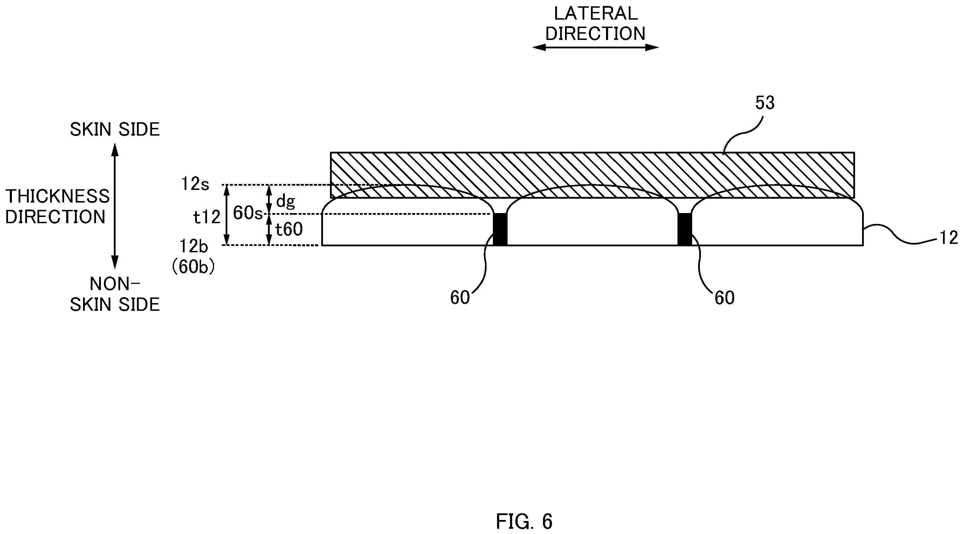

The intermediate fixing portions 53 (adhesive) are provided on the surface of the skin-side sheet 12 having such a plurality of compressed portions 60. FIG. 5 is a schematic cross-sectional view showing a state in which the intermediate fixing portion 53 is provided on the skin-side surface of the skin-side sheet 12 in the diaper 1 of the present embodiment. FIG. 6 is a schematic cross-sectional view showing a state in which the intermediate fixing portion 53 is provided on the skin-side surface of the skin-side sheet 12 in a comparative example. FIG. 5 and FIG. 6 are diagrams showing the joining states of the skin-side sheet 12 and the intermediate fixing portion 53 in the thickness direction, and, for the simplification of description, the leakproof wall portions 40 are not shown.

As described above, the skin-side sheet 12 is compressed in the thickness direction in the compressed portions 60. Therefore, as shown in FIG. 5 and FIG. 6, the thickness t60 of portions in the skin-side sheet 12 where the compressed portions 60 (shown in solid black) are formed is smaller than the thickness t12 of a portion in the skin-side sheet 12 where the compressed portions 60 are not formed. That is, in the thickness direction, skin ends 60s of the compressed portions 60 are positioned on the non-skin side with respect to a skin-side end 12s (skin side surface) of the skin-side sheet 12. Between both ends, a distance dg (= t12 - t60) is provided.

In FIG. 5 and FIG. 6, a non-skin-side end 12b (non-skin side surface) of the skin-side sheet 12 and non-skin-side ends 60b of the compressed portions 60 are at the same position in the thickness direction. However, the non-skin-side ends 60b may be positioned on the skin side with respect to the non-skin side end 12b. That is, a predetermined distance corresponding to the distance dg on the skin side may be provided between the non-skin side end 12b and the non-skin side ends 60b in the thickness direction.

In addition, the intermediate fixing portion 53 indicated by hatched lines is provided on the skin-side surface of the skin-side sheet 12, and the skin-side sheet 12 is impregnated in the thickness direction with a part of the adhesive that forms the intermediate fixing portion 53. Here, the term "to impregnate" refers to dip fabric, paper, or the like into liquid such as a chemical, a resin, or water, causing it to include the liquid. In the present embodiment, as shown in FIG. 5, the skin-side sheet 12 is impregnated with the adhesive that forms the intermediate fixing portion 53, up to a portion where the compressed portions 60 are provided. That is, there are portions where the adhesive and the compressed portions 60 overlap in the thickness direction.

On the other hand, in FIG. 6 of the comparative example, only a portion of the skin-side sheet 12 up to a portion of the distance dg in the thickness direction is impregnated with the adhesive that forms the intermediate fixing portion 53. That is, in the comparative example, the adhesive and the compressed portions 60 do not overlap in the thickness direction, and the adhesive is provided only in regions close to the skin-side surface of the skin-side sheet 12. In this case, the intermediate fixing portion 53 is not capable of obtaining a sufficient joining strength and is likely to peel off from the skin-side sheet 12.

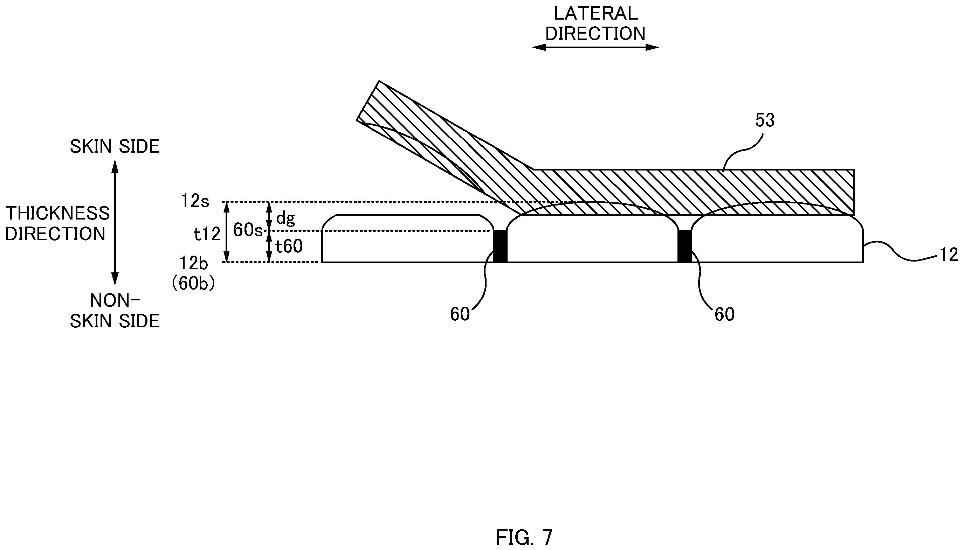

FIG. 7 is a diagram illustrating how the intermediate fixing portion 53 of the comparative example peels off from the skin-side sheet 12. In the comparative example, when a force acts in a direction in which the intermediate fixing portion 53 is peeled off from the skin-side sheet 12, fibers in the surface layer of the skin-side sheet 12 are torn off, and, as in FIG. 7, only the torn fibers in the surface layer detach from the skin-side sheet 12 together with the adhesive that forms the intermediate fixing portion 53. Therefore, the joining strength of the intermediate fixing portion 53 in the comparative example depends on the ease of peeling the fibers in the surface layer of the skin-side sheet 12, and it is difficult to obtain a sufficient joining strength.

In contrast, in the intermediate fixing portion 53 of the present embodiment, the adhesive-impregnated depth reach up to the compressed portions 60. Accordingly, even when a force acts in the direction in which the intermediate fixing portion 53 is peeled off from the skin-side sheet 12, the compressed portions 60 makes it possible to suppress the peeling of fibers. That is, in order to peel off the intermediate fixing portion 53 from the skin-side sheet 12, it is necessary to break the compressed portions 60 and to peel off fibers together with the broken compressed portions 60. Therefore, the joining strength of the intermediate fixing portion 53 in the present embodiment becomes equal to the material breaking strength in the compressed portion 60 of the skin-side sheet 12. Compared with the intermediate fixing portion 53 in the comparative example, it is possible to obtain a sufficiently strong joining strength.

As described above, in the diaper 1 of the present embodiment, since the skin-side sheet 12 is impregnated with the adhesive that forms the intermediate fixing portion 53, up to a position that overlaps the compressed portions 60 in the thickness direction of the skin-side sheet 12. And, the compressed portions 60 in the skin-side sheet 12 and the adhesive are integrally fixed. Accordingly, it is less likely for the intermediate fixing portions 53 to peel off. Therefore, in the region in the vicinity of the crotch portion in which the intermediate fixing portions 53 are provided, even when there is an influence of friction with the body of the wearer or wetting caused by excrement such as urine while the diaper 1 is put on, it becomes easy to maintain the joining between the leakproof wall portions 40 and the absorbent body 10 (skin-side sheet 12).

It should be noted that, in the state of FIG. 5, the adhesive that forms the intermediate fixing portion 53 overlaps the skin-side ends 60s of the compressed portions 60 in the thickness direction, but does not overlap the non-skin-side ends 60b. In other words, the skin-side sheet 12 is impregnated with the adhesive that forms the intermediate fixing portion 53, up to a halfway position of the thickness t60 of the compressed portions 60 in the thickness direction. The compressed portions 60 are not entirely impregnated, that is, the skin-side sheet 12 is not entirely impregnated. This suppresses the skin-side sheet 12 becoming excessively hard due to the adhesive, making it easier to maintain soft texture of the absorbent body 10 (skin-side sheet 12). Therefore, it is possible to make it difficult to cause discomfort to the wearer while the diaper 1 is put on.

Meanwhile, the adhesive that forms the intermediate fixing portion 53 may overlap the entire compressed portions 60 in the thickness direction. That is, the adhesive may overlap the non-skin-side ends 60b of the compressed portions 60. In this case, the compressed portions 60 are integrally fixed throughout the entire thickness t60 with the adhesive, and therefore the joining strength between the intermediate fixing portion 53 and the skin-side sheet 12 becomes stronger. Accordingly, even in a case where a larger load (friction) is applied to the intermediate fixing portions 53 or even in a case where the intermediate fixing portion frequently comes into contact with excrement such as urine, it is easy to maintain the joining between the leakproof wall portions 40 and the absorbent body 10.

In a case where the adhesive overlaps the entire compressed portions 60 in the thickness direction, the skin-side sheet 12 is impregnated with the adhesive up to the non-skin side surface of the skin-side sheet 12 (the non-skin-side end 12b in FIG. 5). At this time, a configuration is also acceptable in which the core wrap sheet 15 is also impregnated with the adhesive. That is, the adhesive may have a portion that overlaps the core wrap sheet 15 in the thickness direction. As shown in FIG. 3, the intermediate fixing portion 53, the skin-side sheet 12, and the core wrap sheet 15 are stacked from the skin side toward the non-skin side in the thickness direction. In this state, in a case where the adhesive-impregnated depth reaches the core wrap sheet 15, the skin-side sheet 12 and the core wrap sheet 15 are in a state of being joined to each other. That is, the core wrap sheet 15 is in a state of being fixed by the skin-side sheet 12. This makes less likely to cause the shape collapse and/or positional deviation of the absorbent core 11 covered with the core wrap sheet 15, making it possible to enhance the fitting property and/or absorption performance of the absorbent body 10.

Even in a case where the core wrap sheet 15 is impregnated with the adhesive, the adhesive-impregnated depth does not reach regions in the leakproof wall portions 40 that are on the non-skin side in the thickness direction with respect to the absorbent core 11 (core wrap sheet 15). That is, the adhesive does not overlap, in the thickness direction, portions 12be where two lateral side portions of the skin-side sheet 12 are folded back toward the non-skin side so as to enclose the absorbent core 11 in FIG. 3. In a case where the adhesive is provided in such a manner that the skin-side sheet 12 is impregnated up to the portions 12be, there is a risk that the adhesive exude laterally outside the portions 12be and adhere to the body or clothing of the wearer. Therefore, preventing the adhesive-impregnated depth from reaching the non-skin side of the absorbent core 11 (core wrap sheet 15) in the thickness direction makes it possible to suppress the exudation of the adhesive.

In addition, the skin-side surfaces of the leakproof wall portions 40 are not impregnated with the adhesive that forms the intermediate fixing portions 53. That is, it is desirable that the adhesive does not overlap the skin-side surfaces of the non-skin-side portions 43 (leakproof wall portions 40) that are stacked on the skin side of the intermediate fixing portions 53 in the thickness direction in FIG. 3. In a case where the adhesive-impregnated depth reaches the skin-side surfaces of the leakproof wall portions 40, there is a risk that the exuded adhesive adhere to the body of the wearer. In the present embodiment, since the sheet member 14 that constitutes the leakproof wall portions 40 is formed of a hydrophobic nonwoven fabric or the like and has a structure that is not easily impregnated with the adhesive, it becomes easy to suppress the adhesive exuding on the skin-side surfaces of the leakproof wall portions 40.

In addition, in the diaper 1 of the present embodiment, as shown in FIG. 5, the lateral length (width) W53 of the intermediate fixing portion 53 is larger than the lateral pitch P60 between the compressed portions 60 provided in the skin-side sheet 12. That is, two or more compressed portions 60 and 60 adjacent to each other in the lateral direction in the skin-side sheet 12 are fixed with the adhesive that forms the intermediate fixing portions 53. Therefore, even in a case where a force acts in a direction in which the intermediate fixing portions 53 are peeled off from the skin-side sheet 12, two or more compressed portions 60 makes it possible to resist the force that peels off the intermediate fixing portions. This can further increase the joining strength between the skin-side sheet 12 and the intermediate fixing portions 53.

In addition, the intermediate fixing portions 53 are disposed at lateral positions apart from the outer ends (the folding positions f3 in FIG. 3) of the leakproof wall portions 40 by a predetermined distance g53o. In other words, in the lateral direction, between the intermediate fixing portion 53 and the outer end of the leakproof wall portion 40, a predetermined gap (g53o) is provided. The provision of such gaps reduces the hardness of the lateral outer ends (edges) of the leakproof wall portions 40 and makes soft the texture of the diaper 1 when coming into contact with the body (groin) of the wearer while the diaper 1 is put on. Therefore, it is possible to realize a more favorable fitting property.

In addition, the intermediate fixing portions 53 are disposed at lateral positions apart from the inner ends (the folding positions f2 in FIG. 3) of the leakproof wall portions 40 by a predetermined distance g53i. In other words, in the lateral direction, between the intermediate fixing portion 53 and the inner end of the leakproof wall portion 40, a gap (g53i) is provided. Therefore, the entire regions of the intermediate fixing portions 53 are covered with the leakproof wall portions 40 (non-skin-side portions 43) from the skin side, making it less likely to expose the adhesive that forms the intermediate fixing portions 53 to the outside. This can suppress the exposed adhesive adhering to the body or clothing of wearers while the diaper 1 is put on.

In addition, at least a part of the intermediate fixing portions 53 is arranged side-by-side in the lateral direction with the narrow portion 11C of the absorbent core 11, and is disposed outside the narrow portion 11C in the lateral direction. That is, in a state in which the diaper 1 is unfolded and stretched in the vertical direction (FIG. 2), the following configuration is preferable: the narrow portion 11C of the absorbent core 11 and at least a part of the intermediate fixing portions 53 are disposed overlapping each other in the front-back direction (the longitudinal direction of the absorbent body 10); and the intermediate fixing portions 53 are disposed outside in the lateral direction with respect to side ends 11es of the absorbent core 11.

In the narrow portion 11C, the absorption capacity of the absorbent core 11 decreases by an amount corresponding to the width narrowing in the lateral direction. Accordingly, the intermediate fixing portions 53 are disposed so as not to overlap the absorbent core 11, suppressing the skin-side surface of the narrow portion 11C from being covered with the leakproof wall portions 40 and the intermediate fixing portions 53. This makes it possible to suppress further decrease of the absorption capacity of the absorbent core 11, making it more likely to maintain the absorption capability of the absorbent core 11. Therefore, it becomes easy to suppress the leakage of excrement such as urine.

### Other embodiments

Hitherto, the embodiment of the present invention has been described, but the above-described embodiment is intended to facilitate the understanding of the present invention and is not intended to limit the interpretation of the present invention. In addition, the present invention can be modified or improved without departing from the scope of the invention as defined in the appended claims, and additionally, it is needless to say that equivalents thereof are included in the present invention.

In the above-described embodiment, a diaper in which the front waist portion 20 and the back waist portion 30 are separate members has been exemplified. However, the front waist portion 20 and the back waist portion 30 may be a single member that is continuous by a crotch portion provided between the front waist portion 20 and the back waist portion 30.

In the above-described embodiment, the leakproof wall portions 40 are formed by folding the sheet member 14. That is, the leakproof wall portions 40 are formed in an integrated manner with the sheet member 14. However, the present invention is not limited thereto. For example, the leakproof wall portions 40 may be formed of a separate sheet member that is different from the sheet member 14.

### [Reference Signs List]

1 diaper (absorbent article), 10 absorbent body, 11 absorbent core, 11C narrow portion, 11es side end, 12 skin-side sheet, 12s end (skin side), 12b end (non-skin side), 12be portion, 13 non-skin-side sheet, 14 sheet member, 15 core wrap sheet, 20 front waist portion, 21 inner-layer sheet, 22 outer-layer sheet, 23 waist elastic member, 30 back waist portion, 31 inner-layer sheet, 32 outer-layer sheet, 33 waist elastic member, 35 extension-portion elastic member, 351 inclined portion, 352 straight portion, 40 leakproof wall portion, 41 leakproof-wall stretching/contracting member (stretching/contracting member), 42 skin-side portion, 43 non-skin-side portion, 51 front fixing portion, 52 back fixing portion, 53 intermediate fixing portion, 60 compressed portion, 60s end (skin side), 60b end (non-skin side), BH waist opening, LH leg opening

## Claims

1. An absorbent article (1) comprising:
an absorbent body (10) including an absorbent core (11) and a hydrophilic skin-side sheet (12) disposed on a skin side with respect to the absorbent core (11); and
a pair of leakproof wall portions (40),
in a state in which the absorbent article (1) is unfolded,
the absorbent body (10) having a front-back direction, a lateral direction, and a thickness direction that intersect each other,
the pair of leakproof wall portions (40) being provided on two lateral sides of the absorbent body (10),
a front fixing portion (51) being provided in a front end portion of the absorbent body (10) and fixing the leakproof wall portions (40) so that the leakproof wall portions (40) are incapable of rising,
a back fixing portion (52) being provided in a back end portion of the absorbent body (10) and fixing the leakproof wall portions (40) so that the leakproof wall portions (40) are incapable of rising,
an intermediate fixing portion (53) being provided between the front fixing portion (51) and the back fixing portion (52) in the front-back direction,
the intermediate fixing portion (53) joining the leakproof wall portion (40) and the absorbent body (10) at an intermediate portion in the front-back direction,
the intermediate fixing portion (53) being provided apart from the front fixing portion (51) and the back fixing portion (52) in the front-back direction,
the intermediate fixing portion (53) fixing the leakproof wall portions (40) to the skin-side sheet (12),
in the intermediate fixing portion (53), a predetermined adhesive being provided between the skin-side sheet (12) and the leakproof wall portions (40),
in the intermediate fixing portion (53), the skin-side sheet (12) being impregnated with at least a part of the adhesive,
the skin-side sheet (12) having a plurality of compressed portions (60) in which the skin-side sheet (12) is compressed in the thickness direction,
a portion in which the adhesive and the compressed portions (60) overlap in the thickness direction is provided.

2. The absorbent article (1) according to claim 1, wherein
in the thickness direction,
the adhesive overlaps a skin-side end (60s) of the compressed portion (60) and
the adhesive does not overlap a non-skin-side end (60b) of the compressed portion (60).

3. The absorbent article (1) according to claim 1, wherein
the adhesive overlaps the entire compressed portion (60) in the thickness direction.

4. The absorbent article (1) according to claim 3, wherein
a core wrap sheet (15) that covers the absorbent core (11) is provided, and
the adhesive has a portion that overlaps the core wrap sheet (15) in the thickness direction.

5. The absorbent article (1) according to claim 3 or 4, wherein
in the thickness direction, the adhesive does not overlap a portion of the skin-side sheet (12) that is disposed on a non-skin side with respect to the absorbent core (11).

6. The absorbent article (1) according to any one of claims 1 to 5, wherein
the adhesive does not overlap skin-side surfaces of the leakproof wall portions (40) in the thickness direction.

7. The absorbent article (1) according to any one of claims 1 to 6, wherein
a lateral width (W53) of the intermediate fixing portion (53) is larger than a lateral pitch (P60) between the plurality of compressed portions (60).

8. The absorbent article (1) according to any one of claims 1 to 7, wherein
in the lateral direction, a predetermined gap (g53o) is provided between an outer end of the intermediate fixing portion (53) and an outer end of the leakproof wall portion (40).

9. The absorbent article (1) according to any one of claims 1 to 8, wherein
in the lateral direction, a predetermined gap (g53i) is provided between an inner end of the intermediate fixing portion (53) and an inner end of the leakproof wall portion (40).

10. The absorbent article (1) according to any one of claims 1 to 9, wherein
the absorbent core (11) has a narrow portion (11C) in a central part in the front-back direction,
the narrow portion (11C) having a lateral width smaller than end portions of the absorbent core (11), and
at least a part of the intermediate fixing portion (53) is arranged side-by-side in the lateral direction with the narrow portion (11C), and is disposed outside the narrow portion (11C) in the lateral direction.

## Patentansprüche

1. Absorbierender Artikel (1), der Folgendes umfasst:
einen Saugkörper (10), der einen Saugkern (11) und eine hydrophile Hautseitenlage (12), die auf einer Hautseite in Bezug auf den Saugkern (11) angeordnet ist, einschließt; und
ein Paar von auslaufsicheren Wandteilen (40),
wobei in einem Zustand, in dem der absorbierende Artikel (1) entfaltet ist,
der Saugkörper (10) eine Vorn-Hinten-Richtung, eine laterale Richtung und eine Dickenrichtung aufweist, die einander schneiden,
das Paar von auslaufsicheren Wandteilen (40) auf zwei lateralen Seiten des Saugkörpers (10) bereitgestellt ist,
ein vorderer fixierender Teil (51) in einem vorderen Endteil des Saugkörpers (10) bereitgestellt ist und die auslaufsicheren Wandteile (40) derart fixiert, dass sich die auslaufsicheren Wandteile (40) nicht aufstellen können,
ein hinterer fixierender Teil (52) in einem hinteren Endteil des Saugkörpers (10) bereitgestellt ist und die auslaufsicheren Wandteile (40) derart fixiert, dass sich die auslaufsicheren Wandteile (40) nicht aufstellen können,
ein dazwischenliegender fixierender Teil (53) zwischen dem vorderen fixierenden Teil (51) und dem hinteren fixierenden Teil (52) in der Vorn-Hinten-Richtung bereitgestellt ist,
wobei der dazwischenliegende fixierende Teil (53) die auslaufsicheren Wandteile (40) und den Saugkörper (10) an einem dazwischenliegenden Teil in der Vorn-Hinten-Richtung verbindet,
der dazwischenliegende fixierende Teil (53) beabstandet von dem vorderen fixierenden Teil (51) und dem hinteren fixierenden Teil (52) in der Vorn-Hinten-Richtung bereitgestellt ist,
der dazwischenliegende fixierende Teil (53) die auslaufsicheren Wandteile (40) an der Hautseitenlage (12) fixiert,
wobei in dem dazwischenliegenden fixierenden Teil (53) ein vorgegebenes Haftmittel zwischen der Hautseitenlage (12) und den auslaufsicheren Wandteilen (40) bereitgestellt ist,
in dem dazwischenliegenden fixierenden Teil (53) die Hautseitenlage (12) mit zumindest einem Teil des Haftmittels durchdrungen ist,
die Hautseitenlage (12) eine Vielzahl von komprimierten Teilen (60) aufweist, in denen die Hautseitenlage (12) in der Dickenrichtung komprimiert ist,
ein Teil, in dem das Haftmittel und die komprimierten Teile (60) in der Dickenrichtung überlappen, bereitgestellt ist.

2. Absorbierender Artikel (1) nach Anspruch 1, wobei
in der Dickenrichtung,
das Haftmittel mit einem Hautseitenende (60s) des komprimierten Teils (60) überlappt und
das Haftmittel nicht mit einem Nicht-Hautseitenende (60b) des komprimierten Teils (60) überlappt.

3. Absorbierender Artikel (1) nach Anspruch 1, wobei
das Haftmittel mit dem gesamten komprimierten Teil (60) in der Dickenrichtung überlappt.

4. Absorbierender Artikel (1) nach Anspruch 3, wobei,
eine Kernumhüllungslage (15), die den Saugkern (11) bedeckt, bereitgestellt ist und
das Haftmittel einen Teil aufweist, der mit der Kernumhüllungslage (15) in der Dickenrichtung überlappt.

5. Absorbierender Artikel (1) nach Anspruch 3 oder 4, wobei
in der Dickenrichtung das Haftmittel nicht mit einem Teil der Hautseitenlage (12) überlappt, der auf einer Nicht-Hautseite in Bezug auf den Saugkern (11) angeordnet ist.

6. Absorbierender Artikel (1) nach einem der Ansprüche 1 bis 5, wobei
das Haftmittel nicht mit Hautseitenoberflächen der auslaufsicheren Wandteile (40) in der Dickenrichtung überlappt.

7. Absorbierender Artikel (1) nach einem der Ansprüche 1 bis 6, wobei
eine laterale Breite (W53) des dazwischenliegenden fixierenden Teils (53) größer ist als ein lateraler Abstand (P60) zwischen der Vielzahl von komprimierten Teilen (60).

8. Absorbierender Artikel (1) nach einem der Ansprüche 1 bis 7, wobei
in der lateralen Richtung ein vorgegebener Zwischenraum (g53o) zwischen einem äußeren Ende des dazwischenliegenden fixierenden Teils (53) und einem äußeren Ende des auslaufsicheren Wandteils (40) bereitgestellt ist.

9. Absorbierender Artikel (1) nach einem der Ansprüche 1 bis 8, wobei
in der lateralen Richtung ein vorgegebener Zwischenraum (g53i) zwischen einem inneren Ende des dazwischenliegenden fixierenden Teils (53) und einem inneren Ende des auslaufsicheren Wandteils (40) bereitgestellt ist.

10. Absorbierender Artikel (1) nach einem der Ansprüche 1 bis 9, wobei
der Saugkern (11) einen schmalen Teil (11C) in einem mittleren Teil in der Vorn-Hinten-Richtung aufweist,
wobei der schmale Teil (11C) eine laterale Breite aufweist, die kleiner ist als Endteile des Saugkerns (11), und
zumindest ein Teil des dazwischenliegenden fixierenden Teils (53) in der lateralen Richtung neben dem schmalen Teil (11C) angeordnet ist und außerhalb des schmalen Teils (11C) in der lateralen Richtung angeordnet ist.

## Revendications

1. Article absorbant (1) comportant :
un corps absorbant (10) comprenant une partie centrale absorbante (11) et une feuille hydrophile côté orienté vers la peau (12) disposée sur un côté orienté vers la peau par rapport à la partie centrale absorbante (11) ; et
une paire de parties formant paroi étanche (40),
dans un état dans lequel l'article absorbant (1) est déplié,
le corps absorbant (10) ayant une direction allant d'avant en arrière, une direction allant dans le sens latéral et une direction allant dans le sens de l'épaisseur qui se croisent les unes les autres,
les parties de la paire de parties formant paroi étanche (40) étant mises en œuvre sur deux côtés latéraux du corps absorbant (10),
une partie de fixation avant (51) étant mise en œuvre dans une partie d'extrémité avant du corps absorbant (10) et fixant les parties formant paroi étanche (40) de telle sorte que les parties formant paroi étanche (40) sont incapables de s'élever,
une partie de fixation arrière (52) étant mise en œuvre dans une partie d'extrémité arrière du corps absorbant (10) et fixant les parties formant paroi étanche (40) de telle sorte que les parties formant paroi étanche (40) sont incapables de s'élever,
une partie de fixation intermédiaire (53) étant mise en œuvre entre la partie de fixation avant (51) et la partie de fixation arrière (52) dans la direction allant d'avant en arrière,
la partie de fixation intermédiaire (53) reliant la partie formant paroi étanche (40) et le corps absorbant (10) au niveau d'une partie intermédiaire dans la direction allant d'avant en arrière,
la partie de fixation intermédiaire (53) étant mise en œuvre de manière séparée par rapport à la partie de fixation avant (51) et à la partie de fixation arrière (52) dans la direction allant d'avant en arrière,
la partie de fixation intermédiaire (53) fixant les parties formant paroi étanche (40) sur la feuille côté orienté vers la peau (12),
dans la partie de fixation intermédiaire (53), un adhésif prédéterminé étant placé entre la feuille côté orienté vers la peau (12) et les parties formant paroi étanche (40),
dans la partie de fixation intermédiaire (53), la feuille côté orienté vers la peau (12) étant imprégnée d'au moins une partie de l'adhésif,
la feuille côté orienté vers la peau (12) ayant une pluralité de parties comprimées (60) dans lesquelles la feuille côté orienté vers la peau (12) est comprimée dans la direction allant dans le sens de l'épaisseur,
une partie dans laquelle l'adhésif et les parties comprimées (60) se chevauchent dans la direction allant dans le sens de l'épaisseur est mise en œuvre.

2. Article absorbant (1) selon la revendication 1, dans lequel
dans la direction allant dans le sens de l'épaisseur,
l'adhésif chevauche une extrémité côté orienté vers la peau (60s) de la partie comprimée (60) et
l'adhésif ne chevauche pas une extrémité côté non orienté vers la peau (60b) de la partie comprimée (60).

3. Article absorbant (1) selon la revendication 1, dans lequel
l'adhésif chevauche toute la partie comprimée (60) dans la direction allant dans le sens de l'épaisseur.

4. Article absorbant (1) selon la revendication 3, dans lequel
une feuille de revêtement de partie centrale (15) qui recouvre la partie centrale absorbante (11) est mise en œuvre, et
l'adhésif a une partie qui chevauche la feuille de revêtement de partie centrale (15) dans la direction allant dans le sens de l'épaisseur.

5. Article absorbant (1) selon la revendication 3 ou la revendication 4, dans lequel
dans la direction allant dans le sens de l'épaisseur, l'adhésif ne chevauche pas une partie de la feuille côté orienté vers la peau (12) qui est disposée sur un côté non orienté vers la peau par rapport à la partie centrale absorbante (11).

6. Article absorbant (1) selon l'une quelconque des revendications 1 à 5, dans lequel
l'adhésif ne chevauche pas les surfaces côté orienté vers la peau des parties formant paroi étanche (40) dans la direction allant dans le sens de l'épaisseur.

7. Article absorbant (1) selon l'une quelconque des revendications 1 à 6, dans lequel
une largeur latérale (W53) de la partie de fixation intermédiaire (53) est supérieure à un pas latéral (P60) entre la pluralité de parties comprimées (60).

8. Article absorbant (1) selon l'une quelconque des revendications 1 à 7, dans lequel
dans la direction allant dans le sens latéral, un espace prédéterminé (g53o) est mis en œuvre entre une extrémité extérieure de la partie de fixation intermédiaire (53) et une extrémité extérieure de la partie formant paroi étanche (40).

9. Article absorbant (1) selon l'une quelconque des revendications 1 à 8, dans lequel
dans la direction allant dans le sens latéral, un espace prédéterminé (g53i) est mis en œuvre entre une extrémité intérieure de la partie de fixation intermédiaire (53) et une extrémité intérieure de la partie formant paroi étanche (40).

10. Article absorbant (1) selon l'une quelconque des revendications 1 à 9, dans lequel
la partie centrale absorbante (11) a une partie étroite (11C) dans une partie se trouvant au centre dans la direction allant d'avant en arrière,
la partie étroite (11C) ayant une largeur latérale inférieure à celle des parties d'extrémité de la partie centrale absorbante (11), et
au moins une partie de la partie de fixation intermédiaire (53) est agencée côte à côte dans la direction allant dans le sens latéral avec la partie étroite (11C), et est disposée à l'extérieur de la partie étroite (11C) dans la direction allant dans le sens latéral.
